# EUROPEAN PATENT APPLICATION

(11) **EP 2 772 187 A1**
(43) Date of publication of application: **03.09.2014**
(21) Application number: 14153776.1
(22) Date of filing: 04.02.2014
(51) Int. Cl.: A61B 5/00, A61B 5/103

(54) **Pressure sensing pad, method of making the same, pressure sensing system, and pressure map display**

(30) Priority: 28.02.2013 US 201361770609 P
(71) Applicant: Hill-Rom Services, Inc., Batesville, IN 47006-9167 (US)
(72) Inventor: Gibson, Luke, Greensburg, IN Indiana 47240 (US); Hoffmaster, James N., Batesville, IN Indiana 47006 (US); Receveur, Timothy J., Guilford, IN Indiana 47022 (US); Sauser, Frank, Cincinnati, OH Ohio 45248 (US); Shannon, Gregory John, Indianapolis, IN Indiana 46239 (US); Williams, Josh, Harrison, OH Ohio 45030 (US); Williamson, Rachel L., Batesville, IN Indiana 47006 (US); Wuebker, Bryan William, Harrison, OH Ohio 45030 (US)
(74) Representative: Findlay, Alice Rosemary

(57) **Abstract**

A pressure sensing pad or mat comprises a piezoresistive layer, a top electrically conductive layer comprising a plurality of electrically conductive top strips extending in a first direction along one side of the piezoresistive layer, a bottom electrically conductive layer comprising a plurality of electrically conductive bottom strips extending in a second direction, nonparallel to the first direction, along the other side of the piezoresistive layer, and top and bottom adhesive layers holding the top and bottom strips against the piezoresistive layer so as to inhibit relative displacement of the strips relative to the piezoresistive layer and relative to each other. Also disclosed are a method of manufacturing the pressure sensor pad, a pressure sensing system that employs the a sensor mat, and a pressure map display for displaying a pressure distribution of an object resting on the pressure sensing mat.

## Description

The subject matter described herein relates to pressure sensing pads or mats, methods of making such pads, a pressure sensing system that uses the pad and a pressure map display for reporting the pressure distribution sensed by the pad. One example application for the pad is to monitor interface pressure between the mattress of a hospital bed and an occupant of the bed.

Occupants of hospital beds may be confined to the bed for a lengthy time, which can increase the risk that the patient will develop pressure ulcers. Even a patient who occupies the bed for a shorter time can develop pressure ulcers if conditions conducive to pressure ulcer devlopment are present. Such conditions include bed linens which become moist due to patient perspiration, or simply a patient's inherent predisposition to develop pressure ulcers.

In order to reduce the risk of pressure ulcers it is desirable to monitor interface pressure, which is the pressure at the patient-mattress interface, and to take corrective action if the interface pressure is excessively high at a particular site on the patient's body and/or if a constant pressure has been exerted on the body site for too long. Other conditions may also indicate the need to take corrective action. Example corrective actions include moving the patient or adjusting the contour of the mattress until the interface pressure is more satisfactorily distributed over the patient's body.

For a bed whose mattress includes inflatable bladders, pressure monitoring may be accompished by measuring the pressure of the fluid (typically air) inside the bladders. The pressure inside the bladders is referred to as intrabladder pressure and varies monotonically with the amount of weight imposed on the bladder. However because the quantity of independent bladders is typically small, such a monitoring technique suffers from lack of adequate resolution. Alternatively, an array of sensors interposed between the patient and the mattress can be employed to measure interface pressure. Such sensor arrays may be in the form of pads or mats with embedded pressure sensors. Despite the existence of such pads practitioners of the art continue to seek ways to improve them.

The present invention may comprise one or more of the following features or combinations thereof.

A pressure sensing pad comprises a piezoresistive layer, a top electrically conductive layer comprising a plurality of electrically conductive top strips extending in a first direction along the top side of the piezoresistive layer and defining one or more top interstrip spaces between each neighboring pair of top strips, a bottom electrically conductive layer comprising a plurality of electrically conductive bottom strips extending in a second direction, nonparallel to the first direction, along the bottom side of the piezoresistive layer and defining a bottom interstrip space between each neighboring pair of bottom strips, and top and bottom adhesive layers holding the respective top and bottom strips against the piezoresistive layer so as to inhibit relative displacement of the strips relative to the piezoresistive layer and relative to each other. In one embodiment the pad also includes a cover that occupies the top and bottom interstrip spaces so as to provide additional electrical insulation between neighboring top strips, to provide additional electrical insulation between neighboring bottom strips, and to provide insulation between the strips and the environment external to the mat.

A method of manufacturing the pressure sensor pad includes the steps of providing a sheet of piezoresistive material having a first side and a second side, arranging a first set of electrically conductive strips along one of the sides so that the strips extend longitudinally in a first direction and are laterally spaced from each other, applying an adhesive to hold the first strips to the piezoresistive sheet, arranging a second set of electrically conductive strips along the other side of the piezoresistive sheet so that the strips extend longitudinally in a second direction which is nonparallel to the first direction and are laterally spaced from each other, and applying an adhesive to hold the second strips to the piezoresistive sheet.

A pressure sensing system comprises a sensor mat comprised of: 1) a first set of electrically conductive strips extending in a first direction with each strip spaced from its neighboring strip or strips, 2) a second set of electrically conductive strips extending in a second direction nonparallel to the first direction with each strip spaced from its neighboring strip or strips such that the first and second strips define a sensor array with sensor nodes at notional intersections of the first and second strips, and 3) a piezoresistive material separating the first and second strips so that electrical resistance at each node is a function of pressure applied at the node. The pressure sensing system also includes a source of electrical excitation connected to the first strips so as to excite the first strips in a predetermined sequence, a detector in communication with the second strips for detecting electrical attributes present at the second strips, and means responsive to the electrical attributes for reporting pressure distribution on the mat.

A pressure map display for displaying a pressure distribution of an object resting on a pressure sensing mat includes cells arranged in a pattern corresponding to pressures exerted on sensor nodes of the pressure sensing mat. Each cell is adapted to display a visually discernible feature that corresponds to a range of pressure at sensing nodes of the mat.

The invention will now be further described by way of example with reference to the accompanying drawings, in which:
FIG. **1** is a schematic side elevation view of a portion of a hospital bed showing a mattress, a pressure sensing pad resting on the mattress, and a spatially distributed load applied to the mattress.
FIG. **2** is a plan view in the direction **2--2** of FIG. **1** showing only selected components of the pressure sensing pad.
FIG**. 3** is an exploded schematic view of the pressure sensing pad showing only selected components.
FIG. **4** is a schematic view of an electrically conductive layer of the pad of FIGS. **1-3** in which electrically conductive strips of the layer comprise an electrically conductive fabric and in which interstrip spaces comprise electrically nonconductive fabric.
FIG. **5** is a schematic plan view of a pressure sensing pad or sensor mat with a portion of the mat magnified to show a sensor node bordered by nonsensing connective zones and dead zones.
FIG. **6** is a schematic plan view of a pressure sensing system showing first and second sets of electrically conductive strips forming a series of sensing nodes, a source of electrical excitation connected to the first strips, a detector in communication with the second strips for detecting electrical attributes present at the second strips, and two examples of means for reporting pressure distribution on the mat.
FIG. **7** is a tabulation showing example relationships between pressure exerted on a sensor node of a pressure sensing mat, electrical resistance of a piezoresistive material at the node, and voltage at an output terminal of the node as a function of time for a given excitation at an input terminal of the node.
FIG. **8** is a view of a visual display comprising discrete cells for displaying the pressure distribution of an object resting on a pressure sensor mat and in which each cell corresponds to one or more pressure sensing nodes of the mat.
FIG. **9** is a visual display in the form of an array of cells each of which corresponds to one or more nodes of a pressure sensor mat in which each cell takes on one of two states as a function of pressure applied at the node or nodes.
FIG. **10** is a view similar to that of FIG. 9 in which the state of each cell depends on the magnitude of pressure exerted on the corresponding sensor node or nodes in comparison to a set of pressure ranges and in which the state associated with each range is signified by a color assigned to the range.
FIG. **11** is a view similar to FIG. **10** in which the display also shows an approximate outline of an object that applies a load to the pressure sensor mat.
FIG. **12** is a view of a visual display in which the visual display displays an outline of at least a portion of an object resting on the mat and also highlights one or more regions within the outline where pressure exerted on the mat violates a predefined threshold.
FIGS. **13-15** are graphs showing electrical resistance at a node of a pressure sensing pad as a function of load applied at the node and wherein the resistance versus load relationship is represented by a nonlinear curve fit (FIGS. **13-14****)** and a linear curve fit (FIG. **15****).**

### SENSING PAD:

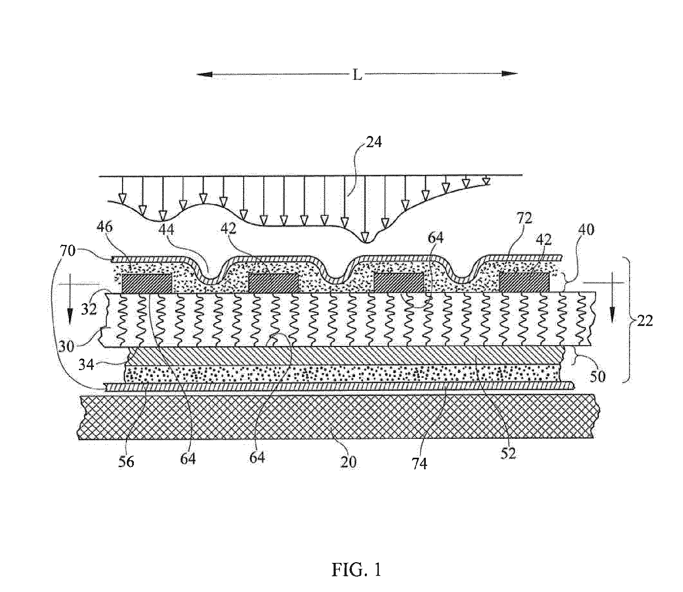

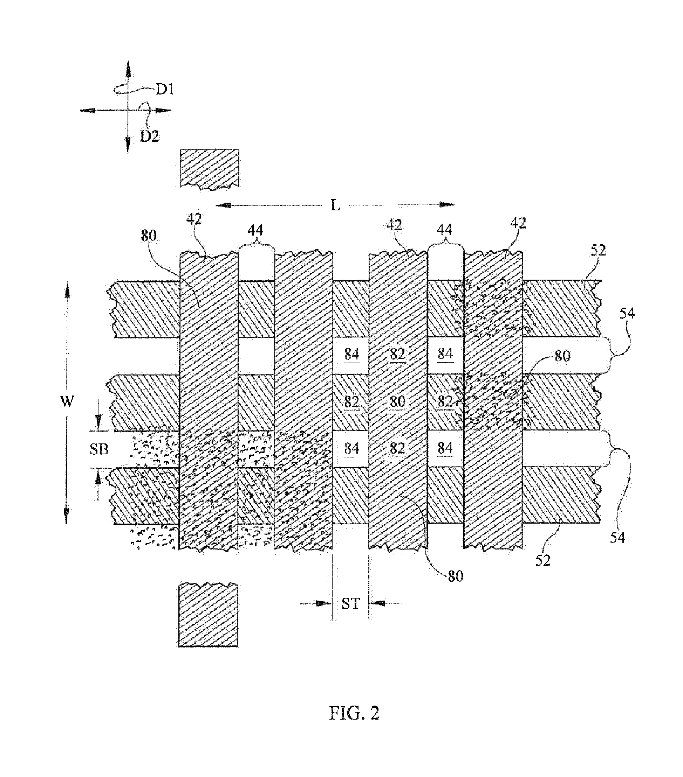

FIGS. **1-2** show a hospital bed mattress **20,** a pressure sensing pad **22** resting on the mattress, and a spatially distributed load **24** supported on the mattress and acting through the pad. In general, and as seen in FIG. **1****,** load **24** varies in a lengthwise direction **L.** In general, load **24** also varies in a widthwise direction **W** which is perpendicular to the plane of FIG. **1** and which is shown in FIG. **2****.** The sensing pad may also be referred to as a sensor pad, a sensing mat or a sensor mat. It should be appreciated that the illustrations in this application are schematic illustrations drawn to promote the reader's understanding of the construction and operation of the mat and that the dimensions of the various pad components and features as depicted are not necessarily in correct proportion to each other or to an actual article.

The sensing pad **22** includes a piezoresistive layer **30** (not shown in FIG. **2****)** having a top side **32** and a bottom side **34.** As used herein the terms "top" and "bottom" do not imply any particular spatial orientation, but instead are used simply to easily distingush between the two sides. One example of a suitable piezoresistive layer is Velostat™, which is a trademark of The 3M Company (formerly known as the Minnesota Mining and Manufacturing Company). In particular, Velostat is a carbon-impregnated polyolefin and is often used to make anti-static packaging for electrical and electronic devices and components. Piezoresistive materials have the property that their electrical resistance decreases with increasing pressure exerted on the material and increases in with decreasing pressure exerted on the material.

Pad **22** also includes a top electrically conductive layer **40** comprising a plurality of electrically conductive top strips **42** extending in a first direction **D1** along the top side **32** of the piezoresistive layer **30** and defining a top interstrip space **44** between each neighboring pair of top strips. The width of the top spaces is **ST.** The pad also includes a bottom electrically conductive layer **50** comprising a plurality of electrically conductive bottom strips **52** extending in a second direction **D2** along bottom side **34** of the piezoresistive layer **30** and defining a bottom interstrip space **54** between each neighboring pair of bottom strips. The width of the bottom spaces is **SB.** Second direction **D2** is nonparallel to first direction **D1**.

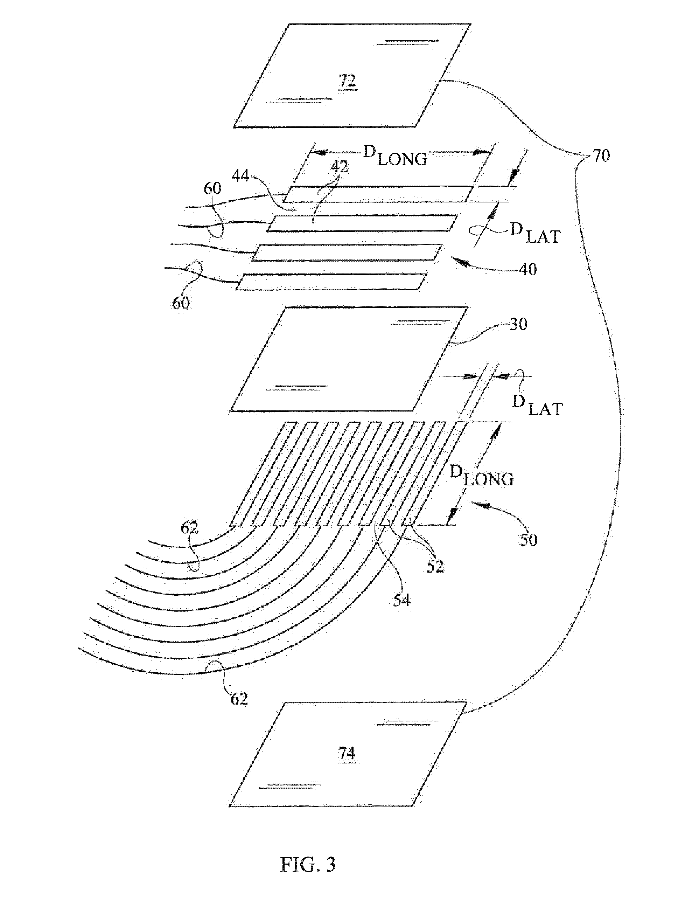

As seen in FIGS. **2-3****,** which shows only electrically conductive strips **42, 52** and patches of an adhesive (shown only in FIG. **2** and described more completely below) strips **42** of top layer **40** are perpendicular to strips **52** of bottom layer **50.** Other nonparallel orientations may also be satisfactory. As seen in FIG. **3** the strips typically have a high aspect ratio (i.e. the ratio of strip longitudinal dimension **D_{LONG}** to strip lateral dimension **D_{LAT}** is much greater than 1.0. As used herein, the terms "longitudinal" and "lateral" refer to the longer and shorter diemnsions respectively of the strips so that the longitudinal direction in one layer (e.g. layer **40)** differs from the longitudinal direction in the other layer (e.g. layer **50).** In the case of top and bottom strips which are perpendicular to each other, the longitudinal direction in one layer is the lateral direction in the other layer. For ease and economy of manufacture of the sensing pad, the strips **42** and **52** have the same lateral dimension **D_{LAT}**, and are uniformly laterally spaced from each other. However architectures involving a nonuniform dimension **D_{LAT}** of strips in one or both of layers **40, 50,** and/or nonuniform interstrip spacing may be satisfactory or even advantageous depending on the particular application in which the sensing pad is to be used. Similarly, strip dimension **D_{LAT}** and interstrip lateral spacing **ST**, **SB** may vary in the longitudinal direction. Even if all the strips have the same lateral dimension **D_{LAT}**, the interstrip spacing **ST** and/or **SB** may be less than, equal to, or greater than **D_{LAT}**. The quantity of strips in the top layer may be equal to or different from the quantity of strips in the bottom layer. As seen in FIG. **3** electrical leads **60, 62** extend from each of the top and bottom strips **42, 52.**

Pad **22** also includes top and bottom electrically nonconductive adhesive layers **46, 56** holding the respective top and bottom electrically conductive strips **42, 52** against the piezoresistive layer **30** to inhibit relative displacement of the strips relative to the piezoresistive layer and relative to each other. As seen best in FIG. **1** each adhesive layer overlies the electrically conductive strips on the same side of the pad and coats the piezoresistive layer in the interstrip spaces **44, 54.** The adhesive layer is substantially continuous in the lengthwise and widthwise directions as suggested by the patch of adhesive depicted in the lower left corner of FIG. **2****.** Alternatively the adhesive may be applied more locally, for example over the electrically conductive strips and extending only a short distance laterally beyond the edges of the strips as suggested by the patches of adhesive depicted in the upper right corner of FIG. **2****.** Either way the adhesive does not and is not intended to be disposed along boundaries **64** between the strips and the piezoresistive layer, although some incidental seepage of adhesive into the boundary near the edges of the strips may be unavoidable during manufacture. The strips are encapsulated between the adhesive and the piezoresistive layer so as to inhibit displacement of the strips relative to the piezoresistive layer and relative to each other.

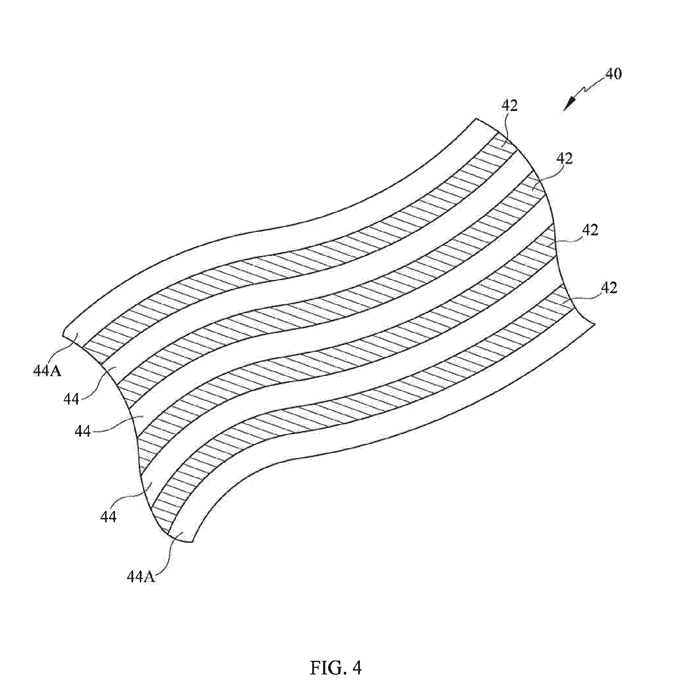

Electrically conductive strips **42, 52** may be metallic or may be strips of electrically conductive fabric. In an embodiment seen in FIG. **4****,** each electrically conductive layer (e.g. layer **40)** is a fabric layer in which the electrically conductive strips (e.g. **42)** are electrically conductive fabric and the interstrip spaces (e.g. **44)** and borders **44A** are electrically nonconductive fabric.

Depending on the exact nature of the specific materials used in manufacture of the pad, the pad may have elastic or stretch properties or may be substantially inelastic or non-stretchable.

Pad **22** may also include an electrically nonconductive cover **70** having a top side or panel **72** that covers top strips **42** and a bottom side or panel **74** that covers bottom strips **52.** As seen in FIG. **1** the cover occupies the interstrip spaces **44, 54** to provide additional insulation between the the strips of the top layer, to provide additional insulation between the strips of the bottom layer, and to provide additional insulation between the strips and the environment external to the pad. In one embodiment the cover is attached to the piezoresistive layer in the interstrip spaces. In the illustrated embodiment the cover is attached to the piezoresistive layer in the interstrip spaces by adhesive layers **46, 56.**

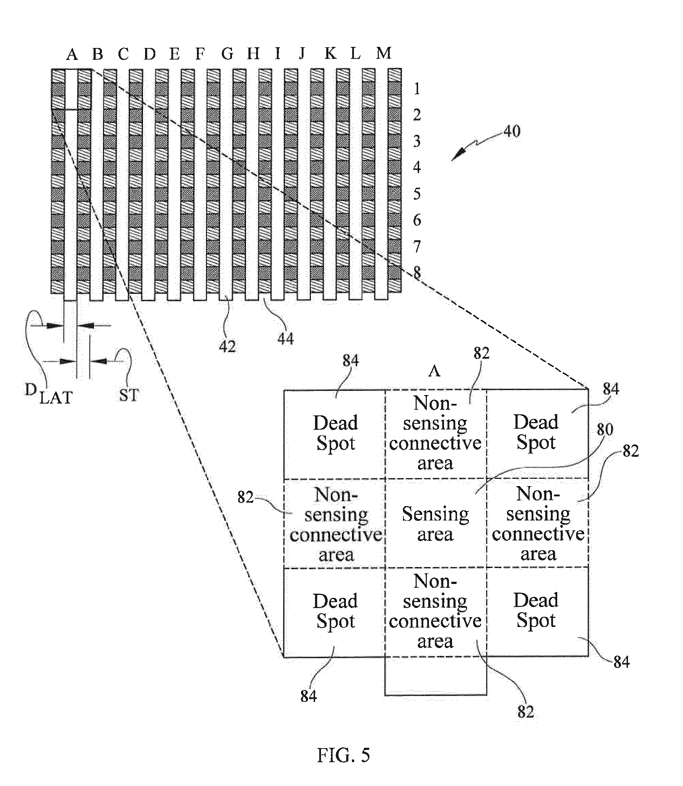

Strips **42, 52** of top and bottom electrically conductive layers **40, 50** cross each other at notional intersections **80.** The intersections are referred to herein as "notional" because the intervening piezoresistive layer **30** prevents actual contact between the strips of one electrically conductive layer and those of the other electrically conductive layer. Each notional intersection is a sensor node and is also designated herein with reference numeral **80.** As seen in FIGS. **2** and **5** each sensor node **80** is bordered at least in part by one or more nonsensing connective zones **82** and one or more dead zones **84.** In FIG. **5** each strip has the identical lateral dimension **D_{LAT}**, and the interstrip spacing **ST** (or **SB)** equals **D_{LAT}**. As a result the sensor nodes **80,** nonsensing connective zones **82** and dead zones **84** are all square and all have the same area. However as already noted such uniformity is not necessary.

If desired, adhesive layers **46, 56** may be absent or substantially absent in at least some of the dead zones **84** as seen in the upper right corner of FIG. **2** where the adhesive layer is shown as extending only slightly beyond the lateral edges of the rightmost conductive strip **42.** In another embodiment cover **70** is absent in at least some of the dead zones. In another embodiment both the adhesive and the cover are absent or substantially absent in the dead zones. When the pad is used in connection with a hospital bed mattress the resulting openings in the pad can transport moisture vapor (perspiration vapor) away from the patient to help keep the patient's skin dry, which reduces the likelihood that the patient will develop pressure ulcers. In addition, the openings will impart additional elasticity or "stretchability" to the pad, which is advantageous if stretchability is desired.

In operation, a load **24** applied to the pad exerts pressure on the piezoresistive layer. In most applications of interest the load, for example the weight of a hospital patient, is a spatially varying load, and the pressure exerted on the piezoresistive layer is a corresponding spatially varying pressure. The resistance of the piezoresistive layer at each node **80** depends on the pressure exerted at that node. The differences in resistance from node to node cause differences in electrical behavior. As explained in more detail below, these differences can be detected and interpreted to reveal how the pressure is distributed on the pad.

### METHOD OF MANUFACTURE

A method of manufacturing a sensor pad can be explained with reference to FIGS. **1-3****.** The method includes providing a sheet of piezoresistive material **30** having a first side **32** and a second side **34,** arranging a first set of electrically conductive strips **42** along one of the sides (e.g. side **32)** so that the strips extend longitudinally in a first direction **D1** and are laterally spaced from each other, and applying an adhesive **46** to hold the first strips **42** to the piezoresistive sheet. The method also includes arranging a second set of electrically conductive strips **52** along the other of the sides (e.g. side **34)** of the piezoresistive sheet so that the strips extend longitudinally in a second direction **D2** which is nonparallel to first direction **D1** and so that strips **52** are laterally spaced from each other. The method also includes applying an adhesive **56** to hold the second strips to the piezoresistive sheet. The piezoresistive sheet with the first and second strips held thereto comprises a subassembly with first and second sides **32, 34.**

The method may also include covering the subassembly with a cover **70.** In one embodiment the cover comprises a first cover panel **72** that is diposed along first side **32** of the subassembly and a second cover panel **74** that is disposed along second side **34** of the subassembly. The first and second cover panels are secured to each other, for example by stitching, at perimeters of the panels to enclose the subassembly. In one variant of the method of manufacture the step of disposing first cover panel **70** along first side **32** is carried out before the adhesive **46** used to hold the first strips **42** to the piezoresistive sheet **30** has cured, and the step of disposing second cover panel **72** along second side **34** is carried out before the adhesive **56** used to hold the second strips to the piezoresistive sheet has cured. As a result when the adhesive cures it holds the cover or cover panels to the piezoresistive material in the interstrip spaces **44, 54.** In a related variant of the method, the step of disposing the first cover panel along the first side of the piezoresistive sheet is followed by smoothing the first cover panel against first side (i.e. against the first side of the piezoresistive sheet and the first conductive strips) and the step of disposing the second cover panel along the second side of the piezoresistive sheet is followed by smoothing the second cover panel against second side (i.e. against the second side of the piezoresistive sheet and the second conductive strips) to help ensure that the first and second cover panels **72, 74** occupy **spaces 44, 46** between neighboring conductive strips and adhere to the piezoresistive sheet.

The above described method yields the construction already described in which notional intersections between the first and second sets of strips each define a sensor node **80** bordered at least in part by one or more nonsensing connective zones **82** and one or more dead zones **84.** The method can be extended to also include removing the dead zones to promote attributes such as vapor transport or stretchability as already mentioned.

The method of manufacture also includes attaching electrical leads **60, 62** to the conductive strips.

The steps of the foregoing method need not be carried out in the order described unless a step is inherently a prerequisite for another step.

### PRESSURE SENSING SYSTEM:

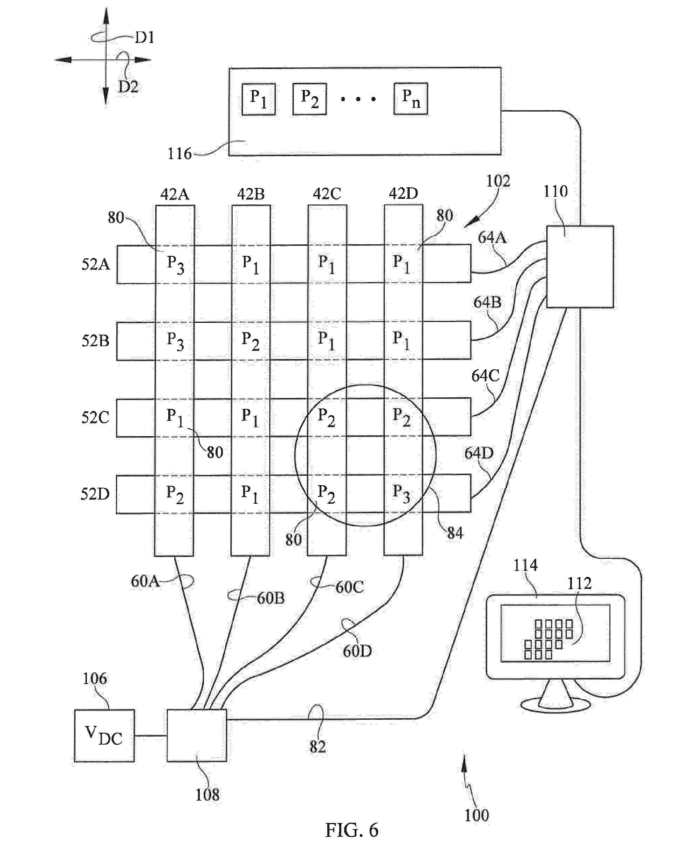

FIG. **6** shows a pressure sensing system **100.** Features similar to or the same as features already described are identified by the same reference numerals already used. The pressure sensing system comprises a sensor mat whose components include a first set of electrically conductive strips **42** extending in a first direction **D1** with each strip laterally spaced from its neighboring strip or strips and a second set of electrically conductive strips **52** extending in a second direction **D2** nonparallel to first direction **D1** with each strip laterally spaced from its neighboring strip or strips such that sensor nodes **80** at notional intersections of the first and second strips define a sensor array **102.** In FIG. **6** only a subset of the sensor nodes is labelled with a reference numeral **80** in order to preserve the clarity of the illustration. The sensor mat components also include a piezoresistive material **30,** not shown in FIG. **6** but visible in FIGS. **1** and **3****.** The piezoresistive material separates the first and second strips from each other. The piezoresistive material causes electrical resistance at each node **80** to be a function of pressure exerted at the node.

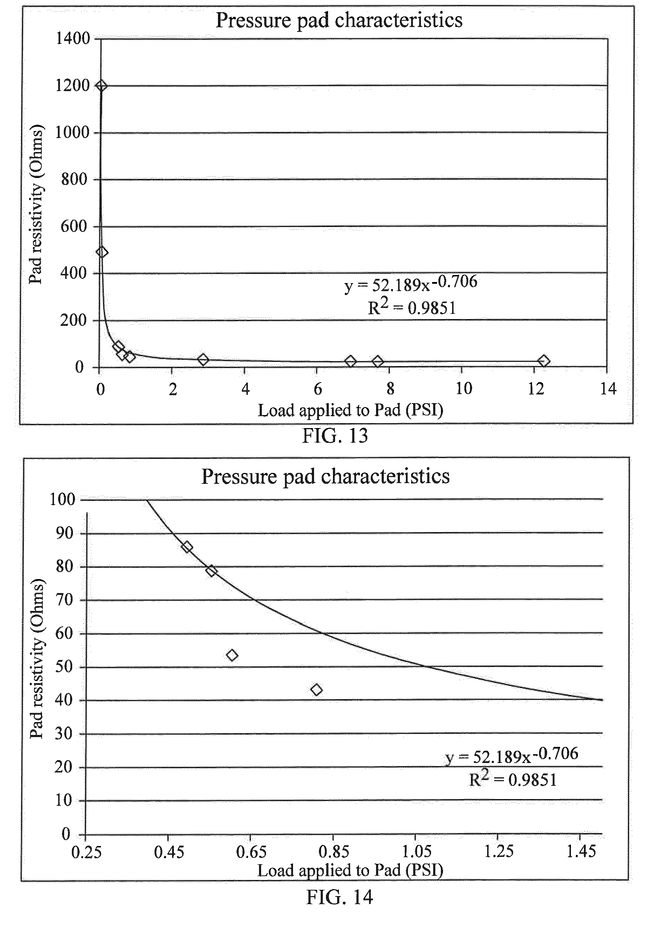

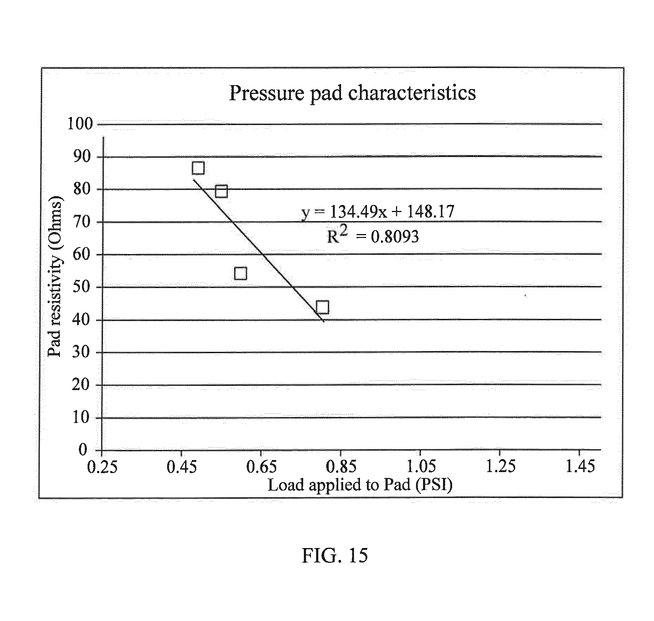

FIGS. **13-14** show a typical calibration curve of electrical resistance at a pressure sensor node **80** as a function of pressure exerted at the node. The data symbols represent experimentally measured data, and the curve is a curve fit through the data. FIG. **13** shows the calibration curve for a range of pressures between about 0 pounds per square inch (psi) to about 12.5 psi; FIG. **14** shows the portion of the curve between about 0.45 and 1.45 psi, which is the range of pressures expected to be exerted on a node by the weight of a patient. FIG. **15** shows a linear fit through the four data points of FIG. **14****.** Each graph also shows the equation of the curve where "x" represents applied pressure and "y" represents resistance.

Pressure sensing system **100** also includes a source of electrical excitation **106** such as 5 volt DC voltage source **V_{DC}** connected to the first strips by leads **60** so that the excitation source can excite the first strips in a predetermined sequence. The system shown in FIG. **6** also includes a switch **108** interposed between the voltage source and top strips **42** for exposing the strips to the voltage source in a predetermined temporal order. In one embodiment the predetermined temporal order is a spatially sequential or succesive order. In other words switch **108** applies 5 volts first to strip **42A,** then to strip **42B,** then to strip **42C** and so forth until all the strips **42** have been excited, after which time switch **108** continues to repeat the same pattern of excitation. The time required to complete an excitation cycle of all the strips **42** is substantially shorter than the speed at which the pressure distribution on mat **22** is expected to change.

The pressure sensing system also includes a detector **110** in communication with second strips **52** for detecting an electrical attribute present at the second strips, and one or more means responsive to the detected electrical attribute for reporting or recording pressure distribution on the mat. Example means for reporting or recording the pressure distribution include a visual display **112** presented on display monitor **114,** and a patient specific record, such as one of the records **Pᵢ,** contained in an electronic medical records database **116.**

In operation switch **108** applies the voltage of the voltage source to strips **42** in the predetermined temporal sequence, for example the switch may apply the voltage to top strip **42A** at time t1, to top strip **42B** at time t2, to top strip **42C** at time t3, etc. as described above. Detector **110** continually detects the voltage present at bottom strips **52** (i.e. at strips **52A, 52B, 52C,** etc.) Because the resistance at each node **80** depends on pressure exerted at the node, the detector will, in general, detect different voltages at any one of strips **52A, 52B, 52C,** etc. at times t1, t2, t3, etc. depending on which strip **42** is being excited by switch **108** at that time. For example, at t1 switch **108** excites strip **42A** and so the voltage detected at strip **52A** indicates the pressure being exerted at node **(52A, 42A),** the voltage detected at strip **52B** indicates the pressure being exerted at node **(52B, 42A),** the voltage detected at strip **52C** indicates the pressure being exerted at node **(52C, 42A)** and so forth. At time t2 switch **108** excites strip **42B** and so the voltage detected at strip **52A** indicates the pressure being exerted at node **(52A, 42B),** the voltage detected at strip **52B** indicates the pressure being exerted at node **(52B, 42B),** the voltage detected at strip **52C** indicates the pressure being exerted at node **(52C, 42B)** and so forth. At later times t3, etc., switch **108** applies the 5 volt excitation to successive top strips **42** and so the voltage detected at each of strips **52** corresponds to the pressure being exerted at the node defined by the excited top strip and each of the bottom strips. Each voltage detected at strips **52** can be converted to a pressure reading by one of the calibration relationships of **FIGS. 13-15** or some other relationship. The pressure distribution on the sensor mat can change over time, for example as a patient adjusts his or her position. However as noted previously the time required to complete an excitation cycle of all the strips **42** is substantially shorter than the speed at which the pressure distribution on mat **22** is expected to change. As a result even though some of the pressure readings are "past values", the collection of pressure readings nevertheless represents an acurate portrayal of the pressure distribution at any given time.

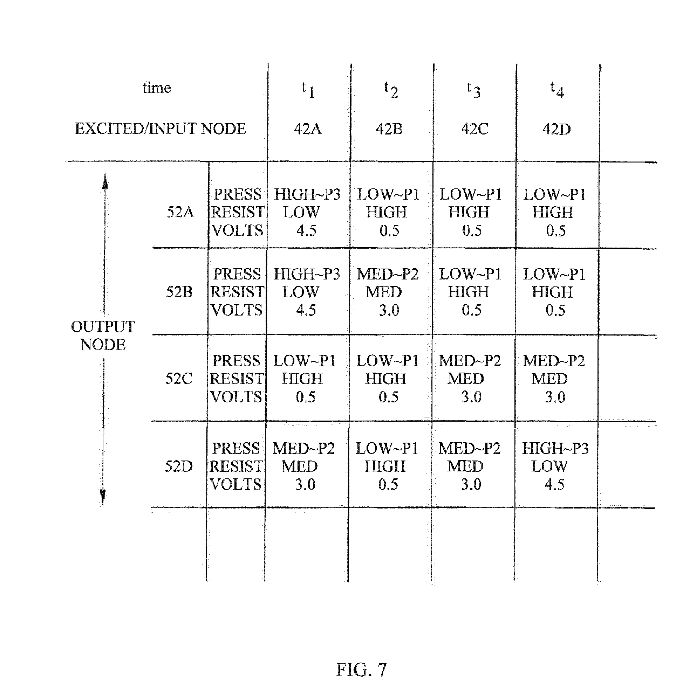

FIG. 7 shows an example in which pressures p1, p2 and p3 are applied at the nodes as indicated on FIG. **6** and where p1 < p2 < p3. At time t1 switch **108** applies 5 volts to strip **42A.** Because the pressure p3 exerted at nodes **(42A, 52A)** and **(42A, 52B)** is high, the resitance at those nodes is low and so detector **110** detects a relatively high voltage (e.g. 4.5 volts) on bottom strips **52A, 52B.** Because the pressure p1 exerted at node **(42A, 52C)** is low, the resitance at that node is high and so detector **110** detects a relatively low voltage (e.g. 0.5 volts) on bottom strips, **52C** at time t1. Because the pressure p2 exerted at node **(42A, 52D)** is between the other two pressures, the resistance at that node is higher than that at nodes **(42A, 52A)** and **(42A, 52B)** but lower than that at node **(42A, 52C).** As a result detector **110** detects an intermediate voltage (e.g. 3.0 volts) on strip **52D** at time t1.

Continuing to refer to FIG. **7****,** at time t2 switch **108** applies 5 volts to strip **42B.** Because the pressure p1 exerted at nodes **(42B, 52A) (42B, 52C)** and **(42B, 52D)is** low, the resistance at those nodes is high and so detector **110** detects a relatively low voltage (e.g. 0.5 volts) on bottom strips **52A, 52C, 52D.** Because the pressure exerted at node **(42B, 52B)** is the moderate pressure p2, the resistance at that node is lower than that at nodes **(42B, 52A) (42B, 52C)** and **(42B, 52D)** and so detector **110** detects a higher voltage (e.g. 3.0 volts) on bottom strip, **52B** at time t2.

At subsequent times t3, t4, etc., switch **108** applies the input excitation voltage to successsive top strips **42** (i.e. **42C, 42D)** and detects output voltage at each of strips **52.** A syncronization signal communicated over communication path **82** (FIG. **6****)** from switch **108** to detector **100** reveals to the detector whether the voltage detected at each of strips **52** is related to the nodes corresponding to top strips **42A, 42B, 42C** or **42D.**

### PRESSURE MAP DISPLAY:

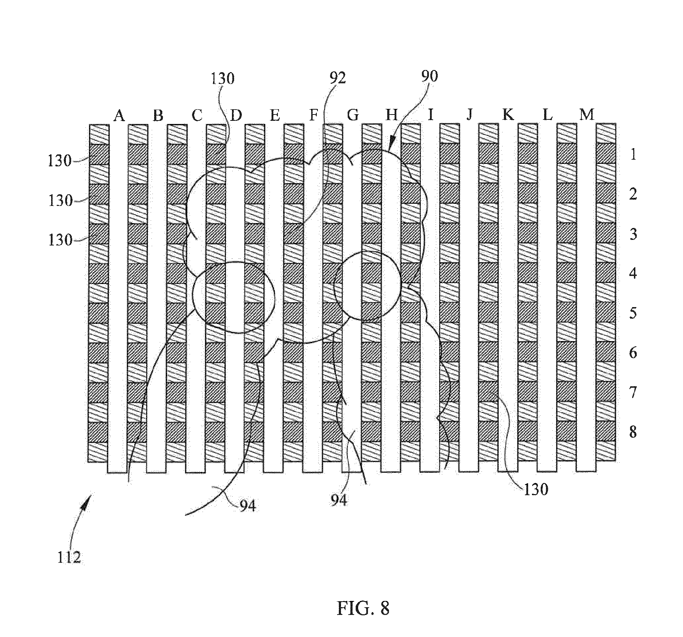

Referring principally to FIGS. **6** and **8****,** one example of a visual display **112** is a pressure map display on display monitor **114** which monitor is adapted to display a pressure distribution of an object resting on a pressure sensing mat **22** having sensing nodes **80.** The display includes discrete cells **130** corresponding to pressure sensing nodes **80** in either a one to one correspondence or some other correspondence (for example a single display cell could display the average of the pressures detected at four nodes in a given quadrant of nodes, e.g. representative quadrant **84** of FIG. 6). In the interest of simplicity, explanations and examples in this application are based on a one to one corrrespondence between cells and nodes. FIG. **8** shows a complete matrix or array of cells and also shows an approximate projection **90** of the buttocks **92** and upper thighs **94** of a patient superimposed on the cellular array. The patient projection **90** of FIG. **8** is shown for reference and is not part of the display itself.

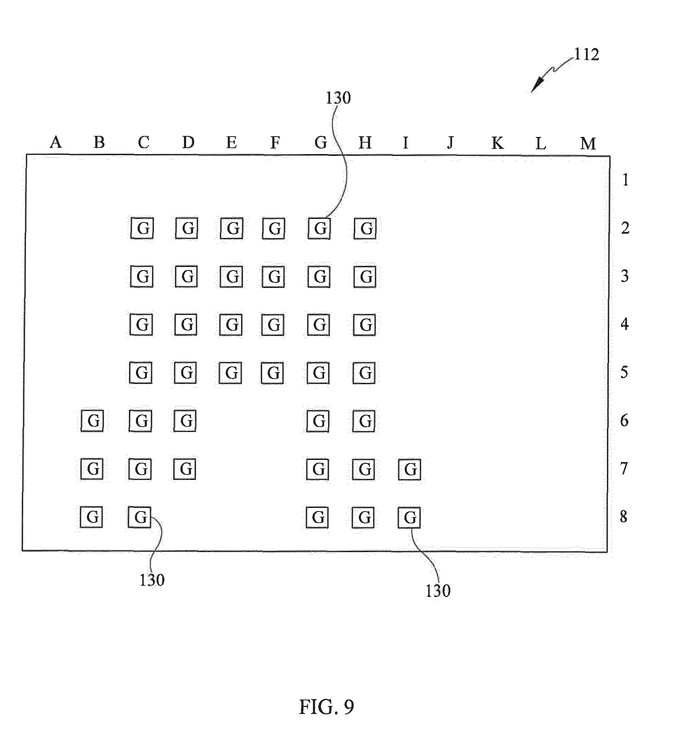

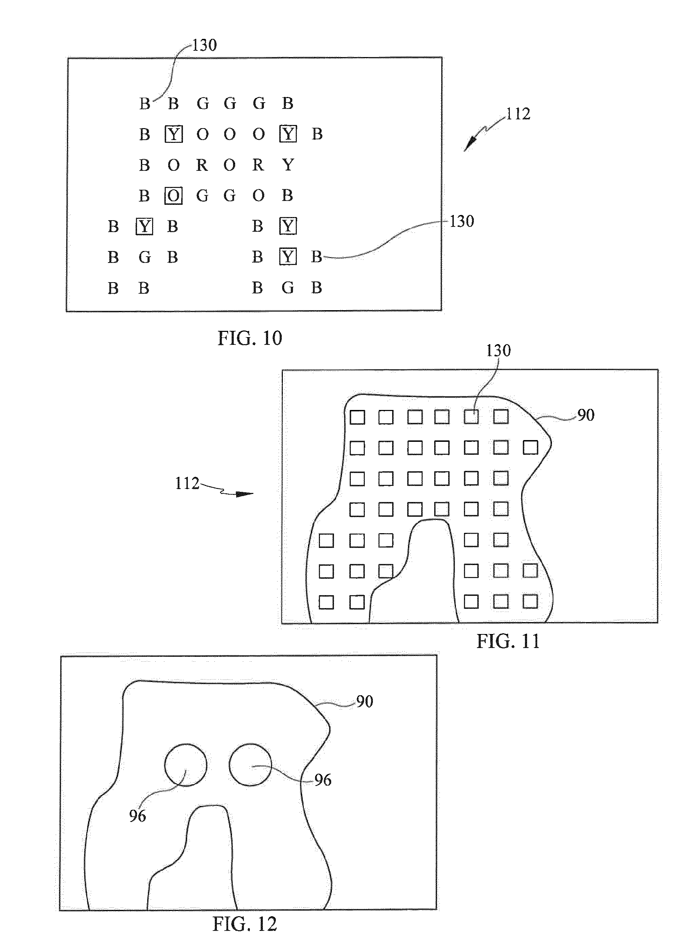

Referring additionally to FIGS. **9-10****,** selected cells **130** are activated to display a visually discernible feature in a pattern that conveys information about the pessure distribution on sensor mat **22.** The pattern of activated cells in FIGS. **9-10** also approximates a projection of at least a portion of an object resting on the mat (e.g. the buttocks and upper thighs of a hospital patient shown superimposed on display **112** of FIG. **8****).** Each cell takes on a state which is a function of the pressure applied at the corresponding node of the pressure sensor mat. For example each cell may display a visually discernible feature, such as a color, related to the magnitude of the pressure exerted on the corresponding node. In one embodiment, shown in FIG. **10****,** each cell takes on one of **N** states, where **N** > **1,** such that each state corresponds to a range of pressure. The following table shows an example in which **N** = 5 and the visually discernible feature is color (indicated on FIG. **10** by letter codes R, O, Y, B, G). In the "pressure range" column of the table "p" is the pressure sensed by a sensor node **80** and PB1, PB2, PB3, and PB4 are boundaries of pressure ranges.

**Table 1**

| Pressure Range | Display Color |
|---|---|
| p ≥ PB4 | Red (R) |
| PB3 ≥ p < PB4 | Orange (O) |
| PB2 ≤ p < PB3 | Yellow (Y) |
| PB1 ≤ p < PB2 | Green (G) |
| p < PB1 | Blue (B) |

As seen in table 2 the same example can also be thought of as having six states, one of which is a null or baseline state. Display cells corresponding to nodes at which the pressure is less than or equal to a minimum pressure boundary PB0 are not activated (e.g. do not display a color). Due to their nonactive or null states, these cells are not individually depicted in FIG. **10****.**

**Table 2**

| Pressure Range | Display Color |
|---|---|
| p ≥ PB4 | Red (R) |
| PB3 ≤ p < PB4 | Orange (O) |
| PB2 ≤ p < PB3 | Yellow (Y) |
| PB1 ≤ p < PB2 | Green (G) |
| PB0 ≤ p < PB1 | Blue (B) |
| p < PB0 | None (null or inactive state) |

Referring to FIG. **9****,** in another specific embodiment **N** = 2 and a cell takes on a state **S1** or **S2** depending on the magnitude of a pressure applied to the corresponding node relative to a pressure threshold value PT. For example a cell may display the color green (state **S2)** if the pressure at the corresponding node is on one side of the threshold value and may take on a different state (state **S1**) if the pressure is equal to or on the other side of the threshold value. In the example seen in FIG. **9** and in table 3, state **S2** is the null state described above, i.e. cells whose corresponding nodes are subject to a pressure equal to or on the other side of the threshhold may remain in a nonactivated state. In the "pressure" column of table 3 "p" is the pressure sensed at a node **80** and PT is the threshhold pressure to which sensed pressure p is compared.

**Table 3**

| Pressure | State | Display Color |
|---|---|---|
| p ≥ PT | S1 | Green (G) |
| p < PT | S2 | None (null or inactive state) |

The example of FIG. **9** can also be thought of as range dependent rather than threshhold dependent as seen in table 4:

**Table 4**

| Pressure Range | State | Display Color |
|---|---|---|
| p ≥ PT | S1 | Green (G) |
| 0 ≤ p < PT | S2 | None (null or inactive state) |

Referring to FIG. **11****,** in another embodiment an approximate outline **90** of the object (patient) exerting pressure on the sensor mat may be displayed along with the activated cells. One possible method for determining the location of the outline is to search for neighboring nodes where the node-to-node pressure gradient is high and one of the pressure magnitudes is small enough to suggest the absence of any load on that node.

Referring to FIG. **12****,** in another embodiment the visual display is adapted to display an outline of an object resting on the mat and to highlight one or more regions **96,** within the outline where pressure exerted on the mat violates a predefined threshold of criticality. The regions shown in the illustration correspond approximately to the cells labelled with color code "R" in FIG. **10****.**

Embodiments of the invention can be described with reference to the following numbered clauses, with preferred features laid out in the dependent clauses:
1. A sensing pad comprising a piezoresistive layer having a top side and a bottom side; a top electrically conductive layer comprising a plurality of electrically conductive top strips extending in a first direction along the top side of the piezoresistive layer and defining a top interstrip space between each neighboring pair of top strips; a bottom electrically conductive layer comprising a plurality of electrically conductive bottom strips extending in a second direction along the bottom side of the piezoresistive layer and defining a bottom interstrip space between each neighboring pair of bottom strips, the second direction being nonparallel to the first direction; top and bottom adhesive layers holding the respective top and bottom strips against the piezoresistive layer so as to inhibit relative displacement of the strips relative to the piezoresistive layer and relative to each other.
2. The pad of clause 1 comprising a cover having a top side that covers the top strips and a bottom side that covers the bottom strips, the cover occupying the interstrip spaces so as to prevent neighboring top strips from contacting each other and to prevent neighboring bottom strips from contacting each other.
3. The pad of clause 2 wherein the cover is attached to the piezoresistive layer in the spaces.
4. The pad of clause 3 wherein the adhesive layer attaches the cover to the piezoresistive layer.
5. The pad of clause 1 wherein the first and second directions are substantially mutually perpendicular.
6. The pad of clause 1 wherein the piezoresistive layer is Velostat™.
7. The pad of clause 1 wherein the adhesive layer is substantially continuous.
8. The pad of clause 1 wherein the electrically conductive strips are strips of electrically conductive fabric.
9. The pad of clause 1 wherein each conductive strip has a lateral dimension and each interstrip space has a lateral dimension smaller than the strip lateral dimension.
10. The pad of clause 1 wherein the pad is substantially nonelastic.
11. The pad of clause 1 wherein each electrically conductive layer is a fabric layer in which the electrically conductive strips are electrically conductive fabric and the interstrip spaces are electrically nonconductive fabric.
12. The pad of clause 1 including electrical leads connected to each conductive strip.
13. The pad of clause 1 wherein notional intersections between the top and bottom strips each define a sensor node bordered at least in part by one or more nonsensing connective zones and one or more dead zones and wherein the adhesive layer is absent in at least some of the dead zones.
14. The pad of clause 2 wherein notional intersections between the top and bottom strips each define a sensor zone bordered at least in part by one or more nonsensing connective zones and one or more dead zones and wherein the adhesive layer and the cover are absent in at least some of the dead zones.
15. A method of making a sensor pad comprising:
   providing a sheet of piezoresistive material having a first side and a second side;
   arranging a first set of electrically conductive strips along one of the sides so that the strips extend longitudinally in a first direction and are laterally spaced from each other;
   applying an adhesive to hold the first strips to the piezoresistive sheet;
   arranging a second set of electrically conductive strips along the other of the sides of the piezoresistive sheet so that the strips extend longitudinally in a second direction which is nonparallel to the first direction and are laterally spaced from each other;
   applying an adhesive to hold the second strips to the piezoresistive sheet.
16. The method of clause 15 wherein the piezoresistive sheet with the first and second strips adhered thereto comprises a subassembly having a first side and a second side and wherein the method comprises covering the subassembly with a cover.
17. The method of clause 15 wherein the piezoresistive sheet with the first and second strips adhered thereto comprises a subassembly having a first side and a second side, and the method comprises:
   disposing a first cover panel along the first side of the subassembly;
   disposing a second cover panel along the second side of the subassembly; and
   securing the first and second cover panels to each other at perimeters thereof to enclose the subassembly.
18. The method of clause 17 wherein the step of disposing the first cover panel along the first side is carried out before the adhesive holding the first strips to the piezoresistive sheet has cured, and the step of disposing the second cover panel along the second side is carried out before the adhesive holding the second strips to the piezoresistive sheet has cured.
19. The method of clause 18 wherein:
   the step of disposing the first cover panel is followed by smoothing the first cover panel against the first side of the piezoresistive sheet and the first set of electrically conductive strips; and
   the step of disposing the second cover panel is followed by smoothing the second cover panel against the second side of the piezoresistive sheet and the second set of electrically conductive strips so that the first and second cover panels occupy spaces between neighboring conductive strips and adhere to the piezoresistive sheet.
20. The method of clause 15 wherein notional intersections between the first and second sets of strips each define a sensor node bordered at least in part by one or more nonsensing connective zones and one or more dead zones and wherein the method includes removing the dead zones.
21. The method of clause 15 including attaching electrical leads to the conductive strips.
22. A pressure sensing system comprising:
   a sensor mat comprised of:
      a first set of electrically conductive strips extending in a first direction with each strip spaced from its neighboring strip or strips,
      a second set of electrically conductive strips extending in a second direction nonparallel to the first direction with each strip spaced from its neighboring strip or strips such that the first and second strips define a sensor array with sensor nodes at notional intersections of the first and second strips; and
      a piezoresistive material separating the first and second strips;
      wherein electrical resistance at each node is a function of pressure applied at the node;
   a source of electrical excitation connected to the first strips so as to excite the first strips in a predetermined sequence;
   a detector in communication with the second strips for detecting electrical attributes present at the second strips; and
   means responsive to the electrical attributes for reporting pressure distribution on the mat.
23. The system of clause 22 wherein the source of electrical excitation is a voltage source.
24. The system of clause 23 including a switch for exposing the strips to the voltage source in a predetermined temporal order.
25. The system of clause 22 wherein the predetermined temporal order is a spatially sequential order.
26. The system of clause 22 wherein the means for reporting is a visual display.
27. The system of clause 26 wherein the visual display is an array of cells each of which corresponds to a node and each cell takes on a state which is a function of the pressure applied at the node.
28. The system of clause 27 wherein each cell takes on one of N states where N > 1, and each state corresponds to a range of pressure.
29. The system of clause 28 wherein one of the states is a null state.
30. The system of clause 28 wherein N = 2 and a cell takes on a state N1 or N2 depending on the magnitude of a pressure applied to the corresponding node relative to a threshold.
31. The system of clause 27 wherein the display includes an outline of an object resting on the mat.
32. The system of clause 26 wherein the visual display is adapted to display an outline of an object resting on the mat and to highlight one or more regions within the outline where pressure exerted on the mat violates a predefined threshold.
33. A pressure map display for a display monitor adapted to display a pressure distribution of an object resting on a pressure sensing mat having sensing nodes, the display including discrete cells arranged in a pattern corresponding to pressures exerted on sensor nodes of a pressure sensor mat, each cell displaying a visually discernible feature that corresponds to a range of pressure.
34. The map display of clause 33 wherein the visually discernible feature is color.
35. The map display of clause 33 wherein the visual display is an array of cells each of which corresponds to a pressure sensing node and each cell takes on a state which is a function of the pressure applied at the node.
36. The map display of clause 33 wherein each cell takes on one of N states where N > 1, and each state corresponds to a range of pressure.
37. The map display of clause 36 wherein one of the states is a null state.
38. The map display of clause 36 wherein N = 2 and a cell takes on a state N1 or N2 depending on the magnitude of a pressure applied to the corresponding node relative to a threshold.
39. The map display of clause 33 wherein the display includes an outline of an object resting on the mat.
40. The map display of clause 35 wherein the visual display is adapted to display an outline of an object resting on the mat and to highlight one or more regions within the outline where pressure exerted on the mat violates a predefined threshold.

## Claims

1. A sensing pad comprising
a piezoresistive layer having a top side and a bottom side;
a top electrically conductive layer comprising a plurality of electrically conductive top strips extending in a first direction along the top side of the piezoresistive layer and defining a top interstrip space between each neighboring pair of top strips;
a bottom electrically conductive layer comprising a plurality of electrically conductive bottom strips extending in a second direction along the bottom side of the piezoresistive layer and defining a bottom interstrip space between each neighboring pair of bottom strips, the second direction being nonparallel to the first direction;
top and bottom adhesive layers holding the respective top and bottom strips against the piezoresistive layer so as to inhibit relative displacement of the strips relative to the piezoresistive layer and relative to each other.

2. The pad of claim **1** comprising a cover having a top side that covers the top strips and a bottom side that covers the bottom strips, the cover occupying the interstrip spaces so as to prevent neighboring top strips from contacting each other and to prevent neighboring bottom strips from contacting each other.

3. The pad of claim **2** wherein the cover is attached to the piezoresistive layer in the spaces.

4. The pad of either claim **2** or claim **3** wherein notional intersections between the top and bottom strips each define a sensor zone bordered at least in part by one or more nonsensing connective zones and one or more dead zones and wherein the adhesive layer and the cover are absent in at least some of the dead zones.

5. The pad of any preceding claim wherein the adhesive layer is substantially continuous.

6. The pad of any preceding claim wherein the electrically conductive strips are strips of electrically conductive fabric.

7. The pad of any preceding claim wherein each conductive strip has a lateral dimension and each interstrip space has a lateral dimension smaller than the strip lateral dimension.

8. The pad of any preceding claim wherein the pad is substantially nonelastic.

9. The pad of any preceding claim wherein each electrically conductive layer is a fabric layer in which the electrically conductive strips are electrically conductive fabric and the interstrip spaces are electrically nonconductive fabric.

10. The pad of any preceding claim wherein notional intersections between the top and bottom strips each define a sensor node bordered at least in part by one or more nonsensing connective zones and one or more dead zones and wherein the adhesive layer is absent in at least some of the dead zones.

11. A pressure sensing system comprising the sensing pad of any preceding claim wherein notional intersections between the top and bottom strips define sensor nodes and wherein electrical resistance at each node is a function of pressure applied at the node, a source of electrical excitation connected to one of the electrically conductive layers for exciting the strips of that layer in a predetermined sequence, a detector in communication with the other electrically conductive layer for detecting electrical attributes present at those strips, and means responsive to the electrical attributes for reporting pressure distribution on the mat.

12. A method of making a sensor pad comprising:
providing a sheet of piezoresistive material having a first side and a second side;
arranging a first set of electrically conductive strips along one of the sides so that the strips extend longitudinally in a first direction and are laterally spaced from each other;
applying an adhesive to hold the first strips to the piezoresistive sheet;
arranging a second set of electrically conductive strips along the other of the sides of the piezoresistive sheet so that the strips extend longitudinally in a second direction which is nonparallel to the first direction and are laterally spaced from each other;
applying an adhesive to hold the second strips to the piezoresistive sheet.

13. The method of claim **12** wherein the piezoresistive sheet with the first and second strips adhered thereto comprises a subassembly having a first side and a second side and wherein the method comprises covering the subassembly with a cover.

14. The method of either claim **12** or claim **13** wherein the piezoresistive sheet with the first and second strips adhered thereto comprises a subassembly having a first side and a second side, and the method comprises:
disposing a first cover panel along the first side of the subassembly;
disposing a second cover panel along the second side of the subassembly; and
securing the first and second cover panels to each other at perimeters thereof to enclose the subassembly.

15. The method of claim **14** wherein the step of disposing the first cover panel along the first side is carried out before the adhesive holding the first strips to the piezoresistive sheet has cured, and the step of disposing the second cover panel along the second side is carried out before the adhesive holding the second strips to the piezoresistive sheet has cured.

16. The method of claim **15** wherein:
the step of disposing the first cover panel is followed by smoothing the first cover panel against the first side of the piezoresistive sheet and the first set of electrically conductive strips; and
the step of disposing the second cover panel is followed by smoothing the second cover panel against the second side of the piezoresistive sheet and the second set of electrically conductive strips so that the first and second cover panels occupy spaces between neighboring conductive strips and adhere to the piezoresistive sheet.
